# EUROPÄISCHE PATENTSCHRIFT

(11) **EP 1 386 601 B1**
(45) Veröffentlichungstag und Bekanntmachung des Hinweises auf die Patenterteilung: **17.10.2007**
(21) Anmeldenummer: 03011280.9
(22) Anmeldetag: 17.05.2003
(51) Int. Cl.: A61K 8/34, A61K 8/37, A61K 8/39, A61K 8/92, A61Q 5/06

(54) **Haarwachsprodukt mit flüssiger oder cremeförmiger Konsistenz**
Hair wax of liquid or creamy consistency
Cire capillaire à consistence liquide ou crémeuse

(30) Priorität: 31.07.2002 DE 10234801
(43) Veröffentlichungstag der Anmeldung: 04.02.2004
(73) Patentinhaber: Wella Aktiengesellschaft, 64295 Darmstadt (DE)
(72) Erfinder: Maillefer, Sarah, 1749 Middes (CH); Rehmann, Andre, 3185 Schmitten (CH); Zenhäusern, Benedikt, 1735 Giffers (CH)

(56) Entgegenhaltungen:
- EP-A- 0 868 898
- WO-A-01/00147
- DE-A- 10 057 353
- DE-A- 19 907 313
- E. W. FLICK: "Cosmetic and toiletry formulations, 2nd ed." 1989 , NOYES PUBLICATION, USA XP002262392 204660 * Seite 26, 216, 218, 366, 378, 399, 420, 433, 475, 625 *
- DATABASE CHEMICAL ABSTRACTS [Online] retrieved from STN Database accession no. 136: 374 565 XP002262394 & JP 2002 145758 A (KOSEI CO., LTD) 22. Mai 2002 (2002-05-22)
- M. SCHLOSSMAN ED.: "The chemistry and manufacture of cosmetics vol.II-Formulating, 3rd ed." 2000 , ALLURED PUB. CORP. , USA XP002262393 * Seite 383 *

## Beschreibung

Gegenstand der Erfindung ist ein Verfahren zum Re-Modellieren der menschlichen Frisur unter Verwendung eines Haarwachsproduktes mit flüssiger oder cremeförmiger Konsistenz enthaltend ein wässriges oder wässrig-alkoholisches Trägermedium, Emulgatoren und Wachse, wobei das Gewichtsverhältnis von Emulgator zu Wachs größer als 1 ist.

Haarwachse sind bekannte Produkte zur Haarbehandlung. Sie finden insbesondere Anwendung, um kurzes bis mittellanges Haar trendgerecht in Form zu bringen und der Frisur Halt, Stand und Festigung sowie Glanz zu verleihen. Auch lassen sich mit Haarwachsen Konturen und Texture in der Frisur erzeugen. Herkömmliche Haarwachse haben eine wachsartig feste Konsistenz und werden üblicherweise in Tiegeln angeboten. Die Anwendung beruht auf folgendem Wirkungsprinzip: Die Entnahme der Produktmasse erfolgt mit den Fingern. Das bei Raumtemperatur feste Wachs wird in den Handflächen verteilt und durch die Handwärme aufgeschmolzen oder zumindest stark erweicht. Durch die Erweichung oder Aufschmelzung wird die Einarbeitung des ansonsten zu festen Wachses in das Haar ermöglicht. Im erweichten oder mehr oder weniger flüssigen Zustand wird das Wachs in das Haar eingearbeitet. Im Haar kühlt es ab und erreicht wieder seine feste Ausgangskonsistenz. Dabei erhärtet es und die gestaltete Frisur erhält Stabilität und Halt sowie häufig einen leichten Wet-Look. Durch dieses Wirkprinzip sind den Produktleistungen der herkömmlichen Stylingwachsprodukte enge Grenzen gesetzt. Damit sich das Wachs gut einarbeiten lässt, darf es bei der Entnahme mit der Hand nicht zu hart sein und der Schmelz- oder Erweichunggspunkt muss in der Nähe der Körpertemperatur liegen. Andererseits läßt sich mit derartigen, weichen Wachsen nur eine mäßige Produktleistung hinsichtlich Stand der Haare, Halt und Volumen der Frisur erreichen. Ausserdem ist die Belastung der Haare vergleichsweise hoch. Mit einer härteren Wachszusammensetzung liesse sich zwar eine bessere Festigung und ein besserer Halt erzielen, aber je härter das Wachs ist, umso härter ist auch die Produktmasse und umso schlechter kann diese verarbeitet und in die Haare eingearbeitet werden.

Die Aufgabe bestand darin, ein Haarwachsprodukt zu entwickeln, welches der Frisur verbesserten Halt und Stand verleiht, dem Haar Formbarkeit und einen natürlichen, seidenmatten Glanz verleiht, welches keine sichtbaren Rückstände bildet und gut auswaschbar ist und wobei gleichzeitig die Produktmasse gut verarbeitbar und gut in das Haar einarbeitbar ist.

Es wurde nun gefunden, dass die Anforderungen erfüllt werden durch Verwendung eines Haarwachsprodukts mit flüssiger oder cremeförmiger Konsistenz auf wässriger oder wässrigalkoholischer Basis mit einem Gehalt an Emulgatoren und Wachsen in einem bestimmten Verhältnis. Gegenstand der Erfindung ist die Verwendung eines Haarwachsprodukts mit flüssiger oder cremeförmiger Konsistenz und mit einem Gehalt an
(A) mindestens 40 Gew.% eines wässrigen oder wässrig-alkoholischen Trägermediums,
(B) mindestens 5 Gew.% mindestens eines Emulgators,
(C) mindestens 2 Gew.% mindestens eines Wachses,
wobei das Gewichtsverhältnis von Emulgator (B) zu Wachs (C) größer als 1 ist und vorzugsweise im Bereich von 1,1 bis 4, besonders bevorzugt im Bereich von 1,5 bis 3 liegt, zum Re-Modellieren einer Frisur.

Erfindungsgemäße Haarwachsprodukte zeichnen sich durch einen besonderen zeitlichen Klebrigkeitsgradienten während der Anwendung aus. Unmittelbar nach dem Aufbringen auf das Haar wird eine relativ hohe Anfangsklebrigkeit erreicht, die nach einiger Zeit (z.B. nach 20 bis 30 Sekunden) nachlässt und sich selbsttätig bis auf eine geringe Restklebrigkeit reduziert. Aufgrund der hohen Anfangsklebrigkeit wird das Stylen der Haare und die Formgebung der Frisur gegenüber herkömmlichen, festen Haarwachsprodukten und gegenüber herkömmlichen Stylingcremes deutlich erleichtert. Eine hohe Klebrigkeit der behandelten Haare wäre, wenn sie dauerhaft ist, allerdings unangenehm und unerwünscht. Durch die selbsttätige Reduzierung der Klebrigkeit wandelt sich das Produkt in seinen Wirkungen in ein nur noch leicht klebendes Wachs um. Die leichte Restklebrigkeit gibt einen angenehmen Griff und bietet den besonderen Vorteil, dass jederzeit ein Ummodellieren der Frisur möglich ist. Weitere Vorteile des erfindungsgemäßen Produktes ist eine problemlose Auswaschbarkeit, ein glänzendes, nicht stumpfes Aussehen der Haare und eine kurze Trocknungszeit.

Produkte mit einem Gehalt an Wachs, Emulgator und Wasser sind zwar aus dem Stand der Technik bekannt. Die besonders vorteilhaften Anwendungseigenschaften des erfindungsgemäßen Produktes wurden aber nur bei bestimmten Einsatzmengen und bestimmten Mengenverhältnissen von Wachs zu Emulgator gefunden, wobei der Emulgator im Überschuss vorliegt. Aus der EP 394 078 A1 sind Haarbehandlungsmittel bekannt mit einem Gehalt an einer Mikrodispersion von Wachsen in Wasser, wobei das Mengenverhältnis Wachs zu Emulgator zwischen 1 und 30 liegt, d.h. die Wachse im Überschuss vorliegen. Ein zu geringer Emulgatorgehalt lässt die Haare sehr stumpf und unerwünscht matt erscheinen.

Unter cremeförmiger Konsistenz wird die typische Konsistenz von Cremes, d.h. von pastösen, wasserhaltigen Zubereitungen auf Basis von Fetten oder Wachsen, Wasser und Emulgatoren verstanden. Unter flüssiger Konsistenz wird die typische Konsistenz von Flüssigkeiten, umfassend die unterschiedlichsten Viskositäten, verstanden, soweit die Zusammensetzung bei Raumtemperatur (25°C) fließfähig ist, d.h. von einer schiefen Ebene (45°) abläuft. Die Begriffe "Wachs", "wachsartig", "wachsförmig" etc. beziehen sich insbesondere auf die Definition für Wachse gemäß Ullmanns Encyklopädie der technischen Chemie, 4. Auflage, Band 24, Seite 3. Demnach sind Wachse bei 20°C knetbar, fest bis brüchig hart, grob- bis feinkristallin, durchscheinend bis opak, jedoch nicht glasartig, über 40°C ohne Zersetzung schmelzend, schon wenig oberhalb ihres Schmelzpunktes verhältnismäßig niedrigviskos, stark temperaturabhängig in Konsistenz und Löslichkeit und unter leichtem Druck polierbar. Ein Haarwachsprodukt ist ein Produkt, mit dem Haare aufgrund eines Gehaltes an mindestens einem Wachs zu einer Frisur geformt werden können.

### Trägermedium (A)

Das Trägermedium (A) ist vorzugsweise in einer Menge von 40 bis 90 Gew.%, besonders bevorzugt von 50 oder von 60 bis 80 Gew.% enthalten. Es kann aus Wasser oder aus einem Gemisch aus Wasser und mindestens einem Alkohol gebildet sein. Geeignete Alkohole sind insbesondere ein- oder mehrwertige C1- bis C5-Alkohole wie z.B. Ethanol, n-Propanol, Isopropanol, Butanole, Pentanole, Ethylenglykol, Propylenglykole, Butylenglykole, Glycerin oder Pentandiole. Bevorzugte einwertige Alkohole sind Ethanol und Isopropanol. Bevorzugte mehrwertige Alkohole sind die Propylenglykole und Glycerin. Wasser ist vorzugsweise in einer Menge von 20 bis 70 Gew.%, besonders bevorzugt von 20 bis 60 Gew.% enthalten. Einwertige Alkohole sind vorzugsweise in einer Menge von 10 bis 50 Gew.%, besonders bevorzugt von 15 bis 40 Gew.% enthalten. Mehrwertige Alkohole sind vorzugsweise in einer Menge von 0 bis 10, besonders bevorzugt von 0,5 bis 10 Gew.%, insbesondere von 1 bis 5 Gew.% enthalten. Durch die Variation der Menge an leichtflüchtigen Alkoholen wie Ethanol oder Isopropanol kann die Trocknungsgeschwindigkeit und damit die Dauer der Phase starker Klebrigkeit gezielt auf das gewünschte Maß eingestellt werden.

Es hat sich gezeigt, dass ein Gehalt an Alkohol nicht nur die Trockenzeit verringert, sondern auch eine Reduzierung der nach Trocknung verbleibenden Restklebrigkeit bewirkt. In einer bevorzugten Ausführungsform enthält das erfindungsgemäße Haarwachsprodukt 20 bis 60 Gew.% Wasser, 10 bis 50 Gew.% mindestens eines einwertigen C2- oder C3-Alkohols, wobei Ethanol bevorzugt ist und 0,5 bis 10 Gew.% mindestens eines mehrwertigen C2- bis C5-Alkohols. Das Gewichtsverhältnis von Wasser zu einwertigem Alkohol liegt vorzugsweise im Bereich von 0,5 bis 5, besonders bevorzugt von 1 bis 3.

### Emulgator (B)

Der Gehalt an Emulgatoren (B) ist vorzugsweise 5 bis 40 Gew.%, besonders bevorzugt 10 bis 30 Gew.% Geeignete Emulgatoren und Emulgatorengemische sind insbesondere solche mit einem HLB-Wert von mindestens 10, vorzugsweise von 10 bis 20, besonders bevorzugt von ca. 13 bis ca. 17. Die Emulgatoren können nichtionisch, anionisch, kationisch oder zwitterionisch sein, wobei nichtionische Emulgatoren bevorzugt werden. Geeignete Emulgatoren sind z.B.
- Ethoxylierte Fettalkohole, Fettsäuren, Fettsäureglyceride oder Alkylphenole, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin
- Anlagerungsprodukte von 5 bis 60 mol Ethylenoxid an Rizinusöl oder an gehärtetes (hydriertes) Rizinusöl.
- Mono-, Di- und/oder Triester der Phosphorsäure mit Anlagerungsprodukten von 2 bis 30 mol Ethylenoxid an C8- bis C22-Fettalkohole
- Fettsäurezuckerester, insbesondere Ester aus Saccharose und ein oder zwei C8- bis C22-Fettsäuren, INCI: Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Polyglycerylfettsäureester, insbesondere aus ein, zwei oder mehreren C8- bis C22-Fettsäuren und Polyglycerin mit vorzugsweise 2 bis 20 Glyceryleinheiten.

In einer besonders bevorzugten Ausführungsform haben die Emulgatoren wachsartige Konsistenz und einen Tropfpunkt oberhalb 25°C.

### Wachs (C)

Der Gehalt an Wachsen (C) liegt vorzugsweise im Bereich von 2,5 bis kleiner 40 Gew.%, besonders bevorzugt von 5 bis kleiner 30 Gew.%. Als Wachs kann prinzipiell jedes im Stand der Technik bekannte Wachs bzw. jeder wachsartige Stoff eingesetzt werden. Hierzu zählen tierische, pflanzliche, mineralische und synthetische Wachse, mikrokristalline Wachse, makrokristalline Wachse, feste Paraffine, Petrolatum, Vaseline, Ozokerit, Montanwachs, Fischer-Tropsch-Wachse, Polyolefinwachse z.B. Polybuten, Bienenwachs, Wollwachs und dessen Derivate wie z.B. Wollwachsalkohole, Candelillawachs, Carnaubawachs, Japanwachs, gehärtete Fette, Fettsäureester und Fettsäureglyceride mit einem Erstarrungspunkt von jeweils oberhalb 40°C, Polyethylenwachse und Silikonwachse. Geeignete Wachse sind Stoffe mit wachsartigen Eigenschaften, insbesondere einem Erstarrungspunkt oberhalb 40°C, vorzugsweise oberhalb 55 °C. Die Nadelpenetrationszahl (0,1 mm, 100 g, 5 s, 25°C; nach DIN 51 579) liegt vorzugsweise im Bereich von 2 bis 70, insbesondere von 3 bis 40. Vorzugsweise ist mindestens ein Wachs enthalten, welches eine Nadelpenetrationszahl kleiner 40, besonders bevorzugt kleiner 20 aufweist. Besonders bevorzugt sind Carnaubawachs und Ceresine mit einer Nadelpenetrationszahl kleiner 20 sowie deren Gemische.

### Zusätzliche Öle (D)

In einer besonderen Ausführungsform, insbesondere zur Herstellung von cremeförmigen Produkten mit verbessertem, weichen Anfühlen, enthält das erfindungsgemäße Haarwachsprodukt zusätzlich mindestens eine bei Raumtemperatur(25°C) flüssige, hydrophobe Substanz (D), insbesondere mindestens ein Öl. Der Gehalt an hydrophober Flüssigkeit (D) ist vorzugsweise von 0,1 bis 20 Gew.%, besonders bevorzugt von 1 bis 10 Gew.%. Bei der hydrophoben Substanz kann es sich um einen leicht flüchtigen oder um einen schwerflüchtigen Stoff handeln, wobei schwerflüchtige Öle bevorzugt sind. Die leicht flüchtigen hydrophoben Stoffe sind bei Raumtemperatur flüssig und weisen einen Siedepunkt im Bereich von vorzugsweise 30 bis 250°C, besonders bevorzugt von 60 bis 220 °C auf. Geeignet sind z.B. flüssige Kohlenwasserstoffe, flüssige cyclische oder lineare Silikone (Dimethylpolysiloxane) oder Gemische der genannten Stoffe. Geeignete Kohlenwasserstoffe sind Paraffine oder Isoparaffine mit 5 bis 14 C-Atomen, besonders bevorzugt mit 8 bis 12 C-Atomen, insbesondere Dodekan oder Isododekan. Geeignete flüssige, leichtflüchtige Silikone sind cyclische Dimethylsiloxane mit 3 bis 8, vorzugsweise 4 bis 6 Si-Atomen, insbesondere Cyclotetradimethylsiloxan, Cyclopentadimethylsiloxan oder Cyclohexadimethylsiloxan. Geeignet sind weiterhin Dimethylsiloxan/Methylalkylsiloxan Cyclocopolymere, z.B. Silicone FZ 3109 von Union Carbide, welches ein Dimethylsiloxan/Methyloctylsiloxan Cyclocopolymer ist. Geeignete flüchtige lineare Silikone weisen 2 bis 9 Si-Atome auf. Geeignet sind z.B. Hexamethyldisiloxan oder Alkyltrisiloxane wie Hexylheptamethyltrisiloxan oder Octylheptamethyltrisiloxan. Die nicht flüchtigen, hydrophoben Öle haben einen Schmelzpunkt von unterhalb 25°C und einen Siedepunkt von über 250 °C, vorzugsweise über 300 °C. Hierfür kann prinzipiell jedes dem Fachmann allgemein bekannte Öl eingesetzt werden. In Frage kommen pflanzliche oder tierische Öle, Mineralöle, Silikonöle oder deren Mischungen. Geeignete Silikonöle sind Polydimethylsiloxane, phenylierte Silikone, Polyphenylmethylsiloxane, Phenyltrimethicone, Poly(C1-C20)-alkylsiloxane, Alkylmethylsiloxane. Geeignet sind weiterhin Kohlenwasserstofföle, z.B. Paraffin- oder Isoparaffinöle, Squalan, Öle aus Fettsäuren und Polyolen, insbesondere Triglyceride von C10- bis C30-Fettsäuren. Geeignete pflanzliche Öle sind z.B. Sonnenblumenöl, Kokosöl, Rizinusöl, Lanolinöl, Jojobaöl, Maisöl, Sojaöl. Besonders bevorzugt sind Kohlenwasserstofföle, insbesondere Mineralöle (Paraffinum liquidum) sowie pflanzliche Öle und Fettsäuretriglyceride.

In einer bevorzugten Ausführungsform enthält das erfindungsgemäße Haarwachsprodukt
(A) 40 bis 90 Gew.% eines wässrig-alkoholischen Trägermediums, wobei Wasser zu 20 bis 60 Gew.%, Ethanol und/oder Isopropanol zu 10 bis 50 Gew.% und zwei- oder dreiwertige C2- oder C3-Alkohole zu 0 bis 10 Gew.% enthalten sind und das Gewichtsverhältnis von Wasser zu Ethanol und/oder Isopropanol im Bereich von 0,5 bis 5 liegt,
(B) 10 bis 30 Gew.% mindestens eines nichtionischen Emulgators,
(C) 5 bis 30 Gew.% mindestens eines Wachses und
(D) 0 bis 20 Gew.% mindestens eines hydrophoben, flüssigen Öls,
wobei das Gewichtsverhältnis von Emulgator (B) zu Wachs (C) im Bereich von 1,5 zu 3 liegt.

Zusätzlich zu den vorstehend genannten Inhaltsstoffen können die erfindungsgemäßen Produkte weitere, übliche kosmetische Zusatzstoffe enthalten:
- Kosmetische Farbstoffe in einer Menge bis zu 6 Gew.%, vorzugsweise 0,1 bis 4 Gew.%, z.B. C.I. Pigment Red 4 (C.I. 12 085), C.I. Pigment Green (C.I. 74 260), und/oder C.I. Vat Blue 4 (C.I. 69 800)
- Perlglanzpigmente in einer Menge von bis zu 25 Gew.%, vorzugsweise 1 bis 20 Gew.%, z.B. solche auf Titandioxid/Mica-Basis
- Parfüm- und Duftstoffe in einer Menge von bis zu 2 Gew.%, vorzugsweise 0,01 bis 1 Gew.%
- Lichtschutz- und Konservierungsmittel in einer Menge von bis zu 1 Gew.%, vorzugsweise 0,01 bis 0,5 Gew.%, z.B. Parahydroxybenzoesäureester, Benzoesäure, Salicylsäure, Sorbinsäure, Mandelsäure, Polyhexamethylen-biguanidhydrochlorid oder Isothiazolinonderivate
- Filmbildende Polymere wie z.B. Polyvinylpyrrolidon oder Vinylpyrrolidon/Vinylacetat Copolymere in einer Menge von bis zu 5 Gew.%, vorzugsweise 0,1 bis 4 Gew.%
- Haarpflegende Zusätze wie z.B. Betain, Panthenol in einer Menge von bis zu 5 Gew.%, vorzugsweise 0,01 bis 4 Gew.%.

### Applikations- und Verpackungsformen

Das erfindungsgemäße Produkt kann je nach Konsistenz in geeigneten Verpackungen wie z.B. Tiegeln, Tuben, Flaschen oder ähnlichen abgefüllt werden. Die Verpackungen können mit einer Pumpvorrichtung, z.B. einem mechanisch betriebenen Pumpspender zur Ausbringung der Produktmasse versehen sein. Die Verpackungen können auch mit einer Vorrichtung zum Verschäumen oder einer Vorrichtung zum Versprühen, z.B. einem mechanisch betriebenen Pumpschäumer bzw. einer mechanisch betriebenen Sprühpumpe versehen sein zur Abgabe der Produktmasse in aufgeschäumter Form oder als Spray, sofern die Konsistenz eine Versprühbarkeit gewährleistet. Als mechanische Pump-, Sprüh- und Schäumvorrichtungen können für das erfindungsgemäße Produkt handelsübliche Pumpen und handelsübliche Schaum- bzw. Sprühköpfe verwendet werden.

Eine besonders bevorzugte Ausführungsform ist ein Produkt zur Haarbehandlung, bei welchem eine erfindunsgemäße Zusammensetzung zusammen mit einem geeigneten Treibmittel in einer druckfesten Verpackung abgefüllt und mit einer Vorrichtung zum Verschäumen (Schaumkopf) versehen ist. Geeignete Treibmittel sind insbesondere verflüssigte Treibgase wie z.B. Propan, n-Butan, Isobutan, fluorierte Kohlenwasserstoffe wie z.B. 1,1-Difluorethan oder 1,1,1,2-Tetrafluorethan oder Dimethylether. Diese Treibgase können einzeln oder im Gemisch eingesetzt werden, z.B. ein Gemisch von Propan und/oder Butan und Dimethylether. Besonders bevorzugt ist ein Gemisch von Propan und Butan. Typische Abfüllverhältnisse liegen im Bereich von ca. 80 bis 98 Gew.% Wirkstoffmischung zu 2 bis 20 Gew.% Treibmittel. Die druckfeste Verpackung kann aus einem beliebigen, für Aerosol Sprüh- oder Schaumprodukte bekanntem Material gefertigt sein. Geeignete Materialien sind insbesondere Metalle wie Aluminium oder Weißblech. Als Schaumköpfe können handelsübliche Schaumköpfe verwendet werden.

### Herstellung

Die erfindungsgemäßen Produkte können hergestellt werden, indem die Wachskomponenten aufgeschmolzen und mit den übrigen Komponenten in das wasserhaltige Trägermaterial einemulgiert werden. Leicht flüchtige Komponenten werden gegebenenfalls nach Abkühlen zugegeben und vermischt. Anschließend wird die noch fließfähige Masse in die gewünschte Verpackung abgefüllt. Im Falle eines treibmittelhaltigen Produktes wird abschließend das Treibgas zugegeben und die Verpackung wird mit einem geeigneten Schaumkopf versehen.

### Anwendungsverfahren

Ein weiterer Gegenstand der Erfindung ist ein Verfahren zum Modellieren einer Frisur, wobei das erfindungsgemäße Haarwachsprodukt auf trockenes oder leicht feuchtes Haar aufgebracht wird und innerhalb der zeitlichen Phase starker Klebrigkeit die gewünschte Frisur erstellt wird. Die Einsatzmenge ist je nach Haarlänge und gewünschtem Effekt eine erbsen- bis haselnussgroße Menge, die vorteilhafterweise vor dem Aufbringen auf das Haar in den Handflächen verrieben wird. Durch die spezielle flüssige oder cremige Konsistenz lässt sich das Produkt gezielt und ohne große Einarbeitung besonders leicht im Haar verteilen. Es gibt dem Haar Stand, Definition, Struktur und trotz der weichen oder fluiden Konsistnz einen starken Halt. Die Haare haben ein angenehmes Aussehen und wirken insbesondere weder stumpf noch ölig glänzend. Das Produkt eignet sich ausgezeichnet zum kreativen Modellieren und Re-Modellieren moderner Stylings.

Ein weiterer Gegenstand der Erfindung ist ein Verfahren zum Re-Modellieren einer Frisur, wobei zunächst eine erste Frisur nach dem oben genannten Verfahren erstellt wird und zu einem späteren Zeitpunkt in der zeitlichen Phase der schwachen Restklebrigkeit die erste Frisur in eine andere Frisur umgewandelt wird. Die nachfolgenden Beispiele sollen den Gegenstand der Erfindung näher erläutern.

### Beispiele

**Beispiel 1 (Mengenangaben in g)**

| | 1A | 1B | 1C | 1D | 1E |
|---|---|---|---|---|---|
| Carnaubawachs | 5 | 10 | 10 | 10 | 15 |
| Cremophor^{®} CO60¹⁾ | 10 | 20 | 20 | 10 | 5 |
| Miwagol^{®} V ²⁾ | - | - | 2,5 | - | - |
| Glycerin (86% ig) | - | - | 2,5 | - | - |
| Ethanol (94,7%ig) | 35 | 35 | 30 | 35 | 50 |
| Wasser | Ad 100 | Ad 100 | Ad 100 | Ad 100 | Ad 100 |

| | | | | | |
|---|---|---|---|---|---|
| 1) INCI-Bezeichnung: PEG-60 Hydrogenated Castor Oil 2) INCI-Bezeichnung: Caprylic/Capric Triglyceride | | | | | |

Die erfindungsgemäßen Rezepturen 1A, 1B und 1C weisen einen Überschuß an Emulgator auf und wurden mit den Rezepturen 1D und 1E in ihren anwendungstechnischen Eigenschaften verglichen.
1A: flüssige Konsistenz; gute, aber abgeschwächte Stylingeigenschaften (Softstyling-Variante)
1B: dickflüssige, viskose Konsistenz; gute Stylingeigenschaften, gute Formbarkeit der Haare, starke Festigung (extra strong Variante)
1C: cremeförmige, angenehm weiche Konsistenz; sehr gute Formbarkeit der Haare, sehr gute Stylingeigenschaften
1D: dickflüssige, viskose, gelartige Konsistenz; zu starke Klebrigkeit
1E: dünnflüssige Emulsion; zu starke Klebrigkeit, stumpfes Aussehen der Haare

### Beispiel 2 Wachs-Creme

- 20 g: PEG-60 Hydrogenated Castor Oil
- 10 g: Carnaubawachs
- 2,5 g: Glycerin (86%ig)
- 2,5 g: Caprylic/Capric Triglyceride
- 0,1 g: d-Panthenol
- q.s.: Konservierungsmittel, W-Filter, Parfüm
- 29 g: Ethanol (94,7%ig)
- ad 100 g: Wasser

### Beispiel 3 Wachs-Creme

- 10 g: PEG-60 Hydrogenated Castor Oil
- 15 g: PEG-40 Hydrogenated Castor Oil, 90%ig
- 10 g: Carnaubawachs
- 2,5 g: Glycerin (86%ig)
- 2,5 g: Caprylic/Capric Triglyceride
- 0,1 g: d-Panthenol
- q.s.: Konservierungsmittel, UV - Filter, Parfüm
- 4 g: Ethanol (94,7%ig)
- ad 100 g: Wasser

**Beispiel 4 Aerosol- Haarwachsschäume**

| | 4A | 4B |
|---|---|---|
| Carnaubawachs | 10 | 10 |
| Cremophor^{®} CO60¹⁾ | 20 | - |
| Sucrose Cocoate (92,5%ig) | - | 5 |
| Polyglyceryl-10 Distearate | - | 5 |
| Ceteareth-20 | - | 5 |
| Oleth-20 | - | 5 |
| Miwagol^{®} V ²⁾ | 2, 5 | - |
| Glycerin (86%ig) | 2,5 | 2,5 |
| Octyldodecanol | - | 2,5 |
| PHB-Propylester | 0,2 | 0,2 |
| PHB-Methylester | 0,2 | 0,2 |
| Benzoesäure | 0,2 | 0,2 |
| d-Panthenol | 0,1 | 0,1 |
| Methoxyzimtsäureoctylester | 0,05 | 0,05 |
| Parfüm | 0,1 | 0,1 |
| Ethanol (94,7%ig) | 19,15 | 29,15 |
| Wasser | Ad 100 | Ad 100 |

Die Zusammensetzungen wurden im Verhältnis von 92 Gew.% Wirkstoffmischung zu 8 Gew.% Propan/Butan (4,8 bar) in einer druckfesten Aluminium-Aerosoldose abgefüllt und mit einem Schaumkopf versehen.

## Patentansprüche

1. Verwendung einer Zusammensetzung mit einem Gehalt an
(A) mindestens 40 Gew.% eines wässrigen oder wässrig-alkoholischen Trägermediums,
(B) mindestens 5 Gew.% mindestens eines Emulgators,
(C) mindestens 2 Gew.% mindestens eines Wachses,
wobei das Gewichtsverhältnis von Emulgator (B) zu Wachs (C) größer als 1 ist,
zum Re-Modellieren einer Frisur.

2. Verwendung nach Anspruch 1, **dadurch gekennzeichnet, dass** das Trägermedium (A) in einer Menge von 40 bis 90 Gew.%, der Emulgator (B) in einer Menge von 5 bis 40 Gew.% und das Wachs (C) in einer Menge von 2,5 bis weniger als 40 Gew.% enthalten ist.

3. Verwendung nach einem der vorhergehenden Ansprüche, **dadurch gekennzeichnet, dass** das Gewichtsverhältnis von Emulgator (B) zu Wachs (C) im Bereich von 1,1 bis 4 liegt.

4. Verwendung nach einem der vorhergehenden Ansprüche, **dadurch gekennzeichnet, dass** das Trägermedium (A) ein Gemisch ist aus Wasser und ein- oder mehrwertigen C1- bis C5-Alkoholen.

5. Verwendung nach Anspruch 4, **dadurch gekennzeichnet, dass** Wasser in dem Haarwachsprodukt in einer Menge von 20 bis 70 Gew.%, mindestens ein einwertiger C2- bis C3-Alkohol in einer Menge von 10 bis 50 Gew.% und mindestens ein mehrwertiger C2- bis C5-Alkohol in einer Menge von 0,5 bis 10 Gew.% enthalten ist.

6. Verwendung nach einem der vorhergehenden Ansprüche, **dadurch gekennzeichnet, dass** das Trägermedium (A) ein Gemisch von Wasser und Ethanol in einem Gewichtsverhältnis im Bereich von 0,5 bis 5 enthält.

7. Verwendung nach einem der vorhergehenden Ansprüche, **dadurch gekennzeichnet, dass** der Emulgator (B) ausgewählt ist aus
a) Anlagerungsprodukten von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
b) C12- bis C22-Fettsäuremono- und -diestern von Anlagerungsprodukten von 1 bis 30 mol Ethylenoxid an Glycerin,
c) Anlagerungsprodukten von 5 bis 60 mol Ethylenoxid an Rizinusöl oder an gehärtetes Rizinusöl,
d) Mono-, Di- und/oder Triestern der Phosphorsäure mit Anlagerungsprodukten von 2 bis 30 mol Ethylenoxid an C8- bis C22-Fettalkohole
oder aus Gemischen dieser Emulgatoren.

8. Verwendung nach einem der vorhergehenden Ansprüche, **dadurch gekennzeichnet, dass** das Wachs (C) ausgewählt ist aus tierischen, pflanzlichen, mineralischen und synthetischen Wachsen, mikrokristallinen Wachsen, makrokristallinen Wachsen, festen Paraffinen, Petrolatum, Vaseline, Ozokerit, Montanwachs, Fischer-Tropsch-Wachsen, Polyolefinwachsen, Bienenwachs, Wollwachs und dessen Derivaten, Candelillawachs, Carnaubawachs, Japanwachs, gehärteten Fetten, Fettsäureestern und Fettsäureglyceriden mit einem Erstarrungspunkt von jeweils oberhalb 40°C und Silikonwachsen.

9. Verwendung nach einem der vorhergehenden Ansprüche, **dadurch gekennzeichnet, dass** in dem Haarwachsprodukt zusätzlich mindestens ein hydrophobes, flüssiges Öl in einer Menge von 0,1 bis 20 Gew.% enthalten ist.

10. Verwendung nach einem der vorhergehenden Ansprüche, **dadurch gekennzeichnet, dass** das Haarwachsprodukt
(A) 40 bis 90 Gew.% eines wässrig-alkoholischen Trägermediums enthält, wobei Wasser zu 20 bis 60 Gew.%, Ethanol und/oder Isopropanol zu 10 bis 50 Gew.% und zwei- oder dreiwertige C2- oder C3-Alkohole zu 0 bis 10 Gew.% enthalten sind und das Gewichtsverhältnis von Wasser zu Ethanol und/oder Isopropanol im Bereich von 0,5 bis 5 liegt und
(B) 10 bis 30 Gew.% mindestens eines nichtionischen Emulgators,
(C) 5 bis 30 Gew.% mindestens eines Wachses und
(D) 0 bis 20 Gew.% an hydrophoben, flüssigen Ölen enthält,
wobei das Gewichtsverhältnis von Emulgator (B) zu Wachs (C) im Bereich von 1,5 zu 3 liegt.

11. Verfahren zum Re-Modellieren einer Frisur, wobei zunächst eine erste Frisur erstellt wird, wobei ein Haarwachsprodukt mit flüssiger oder cremeförmiger Konsistenz auf trockenes oder leicht feuchtes Haar aufgebracht wird und innerhalb der zeitlichen Phase starker Klebrigkeit die gewünschte Frisur erstellt wird und zu einem späteren Zeitpunkt in der zeitlichen Phase schwacher Klebrigkeit die erste Frisur in eine andere Frisur umgewandelt wird, wobei das Haarwachsprodukt eine Zusammensetzung aufweist gemäß einem der Ansprüche 1 bis 10.

## Claims

1. Use of a composition with a content of
(A) at least 40% by weight of an aqueous or aqueous-alcoholic carrier medium,
(B) at least 5% by weight of at least one emulsifier,
(C) at least 2% by weight of at least one wax,
where the weight ratio of emulsifier (B) to wax (C) is greater than 1,
for remodelling a hairstyle.

2. Use according to Claim 1, **characterized in that** the carrier medium (A) is present in an amount of from 40 to 90% by weight, the emulsifier (B) is present in an amount of from 5 to 40% by weight and the wax (C) is present in an amount of from 2.5 to less than 40% by weight.

3. Use according to one of the preceding claims, **characterized in that** the weight ratio of emulsifier (B) to wax (C) is in the range from 1.1 to 4.

4. Use according to one of the preceding claims, **characterized in that** the carrier medium (A) is a mixture of water and mono- or polyhydric C1- to C5-alcohols.

5. Use according to Claim 4, **characterized in that** water is present in the hair wax product in an amount of from 20 to 70% by weight, at least one monohydric C2-C3-alcohol is present in an amount of from 10 to 50% by weight and at least one polyhydric C2-C5-alcohol is present in an amount of from 0.5 to 10% by weight.

6. Use according to one of the preceding claims, **characterized in that** the carrier medium (A) comprises a mixture of water and ethanol in a weight ratio in the range from 0.5 to 5.

7. Use according to one of the preceding claims, **characterized in that** the emulsifier (B) is selected from
a) addition products of from 2 to 30 mol of ethylene oxide and/or 1 to 5 mol of propylene oxide onto C8- to C22-fatty alcohols, onto C12-to C22-fatty acids or onto alkylphenols having 8 to 15 carbon atoms in the alkyl group,
b) C12- to C22-fatty acid mono- and diesters of addition products of from 1 to 30 mol of ethylene oxide onto glycerol,
c) addition products of from 5 to 60 mol of ethylene oxide onto castor oil or onto hydrogenated castor oil,
d) mono-, di- and/or triesters of phosphoric acid with addition products of from 2 to 30 mol of ethylene oxide onto C8- to C22-fatty alcohols
or from mixtures of these emulsifiers.

8. Use according to one of the preceding claims, **characterized in that** the wax (C) is selected from animal, vegetable, mineral and synthetic waxes, microcrystalline waxes, macrocrystalline waxes, solid paraffins, petrolatum, vaseline, ozokerite, montan wax, Fischer-Tropsch waxes, polyolefin waxes, beeswax, wool wax and derivatives thereof, candelilla wax, carnauba wax, Japan wax, hydrogenated fats, fatty acid esters and fatty acid glycerides with a solidification point of in each case above 40°C and silicone waxes.

9. Use according to one of the preceding claims, **characterized in that** at least one hydrophobic, liquid oil is additionally present in the hair wax product in an amount of from 0.1 to 20% by weight.

10. Use according to one of the preceding claims, **characterized in that** the hair wax product
(A) comprises 40 to 90% by weight of an aqueous-alcoholic carrier medium, where water is present to 20 to 60% by weight, ethanol and/or isopropanol are present to 10 to 50% by weight and di- or trihydric C2- or C3-alcohols are present to 0 to 10% by weight, and the weight ratio of water to ethanol and/or isopropanol is in the range from 0.5 to 5 and
(B) 10 to 30% by weight of at least one nonionic emulsifier,
(C) 5 to 30% by weight of at least one wax and
(D) 0 to 20% by weight of hydrophobic, liquid oils,
where the weight ratio of emulsifier (B) to wax (C) is in the range from 1.5 to 3.

11. Method for remodelling a hairstyle, where initially a first hairstyle is created, where a hair wax product of liquid or creamy consistency is applied to dry or slightly damp hair and within the time phase of strong stickiness the desired hairstyle is created and at a later time point in the time phase of weak stickiness the first hairstyle is converted to another hairstyle, where the hair wax product has a composition according to one of Claims 1 to 10.

## Revendications

1. Utilisation d'une composition ayant un teneur
(A) d'au moins 40 % en poids en un véhicule aqueux ou aqueux-alcoolique,
(B) d'au moins 5 % en poids en au moins un émulsifiant,
(C) d'au moins 2 % en poids en au moins une cire;
le rapport pondéral de l'émulsifiant (B) à la cire (C) étant supérieur à 1,
pour le remodelage d'une coiffure.

2. Utilisation selon la revendication 1, **caractérisée en ce que** le véhicule (A) est contenu en une quantité de 40 à 90 % en poids, l'émulsifiant (B) est contenu en une quantité de 5 à 40 % en poids et la cire (C) est contenue en une quantité de 2,5 à moins de 40 % en poids.

3. Utilisation selon l'une quelconque des revendications précédentes, **caractérisée en ce que** le rapport pondéral de l'émulsifiant (B) à la cire (C) se situe dans la plage allant de 1,1 à 4.

4. Utilisation selon l'une quelconque des revendications précédentes, **caractérisée en ce que** le véhicule (A) est un mélange d'eau et d'alcools en C₁-C₅ mono- ou polyhydriques.

5. Utilisation selon la revendication 4, **caractérisée en ce que** le produit capillaire cireux contient de l'eau en une quantité de 20 à 70 % en poids, au moins un alcool en C₂-C₃ monohydrique en une quantité de 10 à 50 % en poids et au moins un alcool en C₂-C₅ polyhydrique en une quantité de 0,5 à 10 % en poids.

6. Utilisation selon l'une quelconque des revendications précédentes, **caractérisée en ce que** le véhicule (A) contient un mélange d'eau et d'éthanol en un rapport pondéral dans la plage de 0,5 à 5.

7. Utilisation selon l'une quelconque des revendications précédentes, **caractérisée en ce que** l'émulsifiant (B) est choisi parmi
a) des produits de fixation par addition de 2 à 30 moles d'oxyde d'éthylène et/ou de 1 à 5 moles d'oxyde de propylène sur des alcools gras en C₈-C₂₂, sur des acides gras en C₁₂-C₂₂ ou sur des alkylphénols ayant de 8 à 15 atomes de carbone dans le groupe alkyle,
b) des mono- et diesters d'acides gras en C₁₂-C₂₂ de produits de fixation par addition de 1 à 30 moles d'oxyde d'éthylène sur le glycérol,
c) des produits de fixation par addition de 5 à 60 moles d'oxyde d'éthylène sur l'huile de ricin ou sur l'huile de ricin hydrogénée,
d) des mono-, di- et/ou triesters de l'acide phosphorique avec des produits de fixation par addition de 2 à 30 moles d'oxyde d'éthylène sur des alcools gras en C₈-C₂₂,
ou parmi des mélanges de ces émulsifiants.

8. Utilisation selon l'une quelconque des revendications précédentes, **caractérisée en ce que** la cire (C) est choisie parmi des cires animales, des cires végétales, des cires minérales et des cires synthétiques, des cires microcristallines, des cires macrocristallines, des paraffines solides, le petrolatum, la vaseline, l'ozokérite, la cire de lignite, les cires de Fischer-Tropsch, les cires polyoléfiniques, la cire d'abeille, la lanoline et ses dérivés, la cire de candelilla, la cire de carnauba, la cire du Japon, des graisses durcies, des esters d'acides gras et des glycérides d'acides gras ayant chacun un point de solidification supérieur à 40 °C et des cires de silicone.

9. Utilisation selon l'une quelconque des revendications précédentes, **caractérisée en ce qu'**au moins une huile liquide hydrophobe est contenue en une quantité de 0,1 à 20 % en poids dans le produit capillaire cireux.

10. Utilisation selon l'une quelconque des revendications précédentes, **caractérisée en ce que** le produit capillaire cireux contient
(A) de 40 à 90 % en poids d'un véhicule aqueux-alcoolique, de l'eau étant contenue à raison de 20 à 60 % en poids, de l'éthanol et/ou de l'isopropanol étant contenu (s) à raison de 10 à 50 % en poids et des alcools en C₂ ou C₃ di-
ou trihydriques étant contenus à raison 0 à 10 % en poids et le rapport pondéral de l'eau à l'éthanol et/ou l'isopropanol se situant dans la plage allant de 0,5 à 5 et
(B) de 10 à 30 % en poids d'au moins un émulsifiant non ionique,
(C) de 5 à 30% en poids d'au moins une cire, et
(D) de 0 à 20 % en poids d'huiles liquides hydrophobes,
le rapport pondéral de l'émulsifiant (B) à la cire (C) se situant dans la plage allant de 1,5 à 3.

11. Procédé pour le remodelage d'une coiffure, dans lequel on élabore d'abord une première coiffure en appliquant sur les cheveux secs ou légèrement humides un produit capillaire cireux à consistance liquide ou crémeuse et en l'espace de la phase temporelle de forte pégosité on élabore la coiffure désirée et à un instant ultérieur dans la phase temporelle de faible pégosité on transforme la première coiffure en une autre coiffure, le produit capillaire cireux ayant une composition selon l'une quelconque des revendications 1 à 10.
